# EUROPEAN PATENT APPLICATION

(11) **EP 0 725 079 A1**
(43) Date of publication of application: **07.08.1996**
(21) Application number: 96300613.5
(22) Date of filing: 29.01.1996
(51) Int. Cl.: C07K 14/47

(54) **Anti-obesity proteins**

(30) Priority: 31.01.1995 US 381048; 06.02.1995 US 383638; 22.06.1995 US 450; 11.08.1995 US 2161
(71) Applicant: ELI LILLY AND COMPANY, Indianapolis, Indiana 46285 (US)
(72) Inventor: Basinski, Margret B., Indianapolis, Indiana 46219 (US); Dimarchi, Richard D., Carmel, Indiana 46033 (US); Flora, David B., Greenfield, Indiana 46140 (US); Hale, John E., Fishers, Indiana 46038 (US); Heath Jr., William F., Fishers, Indiana 46038 (US); Hoffmann, James A., Greenwood, Indiana 46143 (US); Schoner, Brigitte E., Monrovia, Indiana 46157 (US)
(74) Representative: Tapping, Kenneth George

(57) **Abstract**

The present invention provides anti-obesity proteins, which when administered to a patient regulate fat tissue. Accordingly, such agents allow patients to overcome their obesity handicap and live normal lives with much reduced risk for type II diabetes, cardiovascular disease and cancer. The peptides are represented by mentioned DNA string or by analogs thereof:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

## Description

The present invention is in the field of human medicine, particularly in the treatment of obesity and disorders associated with obesity. Most specifically the invention relates to anti-obesity proteins that when administered to a patient regulate fat tissue.

Obesity, and especially upper body obesity, is a common and very serious public health problem in the United States and throughout the world. According to recent statistics, more than 25% of the United States population and 27% of the Canadian population are overweight. Kuczmarski, Amer. J. of Clin. Nutr. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately, these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a pharmacological agent that can correct this adiposity handicap and allow the physician to successfully treat obese patients in spite of their genetic inheritance is needed.

Physiologists have postulated for years that, when a mammal overeats, the resulting excess fat signals to the brain that the body is obese which, in turn, causes the body to eat less and burn more fuel. G. R. Hervey, Nature 227: 629-631 (1969). This "feedback" model is supported by parabiotic experiments, which implicate a circulating hormone controlling adiposity.

The *ob /ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed a gene coding for a 167 amino acid protein with a 21 amino acid signal peptide that is exclusively expressed in adipose tissue. Likewise, Murakami et al., in Biochemical and Biophysical Research Communications 209(3):944-52 (1995) report the cloning and expression of the rat obese gene. The protein, which is apparently encoded by the *ob* gene, is now speculated to be an adiposity regulating hormone. No pharmacological activity is reported by Zhang et al.

However, we have discovered that the proteins disclosed by Zhang et al. are poor pharmacological agents due to chemical and/or physical instability. The human protein, for example, is more prone to precipitation. Pharmaceutical formulations of the natural protein containing a precipitate increase the risk of producing an immunological response in the patient. Accordingly, there remains a need to develop pharmacological agents that provide improved physical and chemical stability and that are useful to help patients regulate their appetite and metabolism.

Most significantly, it has now been determined that specific substitutions to amino acid residues 77, 97 to 111, 118, and/or 138 of the human obesity protein lead to a superior therapeutic agent with improved stability. Accordingly, the present invention provides biologically active obesity proteins. The proteins of the present invention are more readily formulated and stored. Furthermore, the present compounds are more pharmaceutically elegant, which results in superior delivery of therapeutic doses. Thus, such agents allow patients to overcome their obesity handicap and live normal lives with a more normalized risk for type II diabetes, cardiovascular disease and cancer.

### Summary of Invention

The present invention is directed to a protein of the Formula (I): 
(SEQ ID NO: 1)
Val Pro Ile Xaa Lys Val Xaa Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Xaa Lys Val Thr Gly Leu Asp Phe Ile Pro Gly Leu His Pro Ile
        35                  40                  45

Leu Thr Leu Ser Lys Xaa Asp Xaa Thr Leu Ala Val Tyr Xaa Xaa Ile
    50                  55                  60

Leu Thr Ser Xaa Pro Ser Arg Xaa Val Ile Xaa Ile Xaa Xaa Asp Leu
65                  70                  75                  80

Glu Xaa Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Xaa Tyr Ser Thr Glu Val Val Ala Leu Ser Arg
        115                 120                 125

Leu Xaa Gly Ser Leu Xaa Asp Xaa Leu Trp Xaa Leu Asp Leu Ser Pro
    130                 135                 140

145
Gly Cys                                                         (I)
 wherein:
Xaa at position 4 is Gln or Glu;
Xaa at position 7 is Gln or Glu;
Xaa at position 22 is Asn, Asp or Glu;
Xaa at position 27 is Thr or Ala;
Xaa at position 28 is Gln, Glu, or absent;
Xaa at position 34 is Gln or Glu;
Xaa at position 54 is Met, methionine sulfoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Asn, Asp or Glu;
Xaa at position 75 is Gln or Glu;
Xaa at position 77 is Ser or Ala;
Xaa at position 78 is Gln, Asn, or Asp;
Xaa at position 82 is Gln, Asn, or Asp;
Xaa at position 118 is Gly or Leu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 139 is Gln or Glu; said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

The invention further provides a method of treating obesity, which comprises administering to a mammal in need thereof a protein of the Formula (I).

The invention further provides a pharmaceutical formulation, which comprises a protein of the Formula (I) together with one or more pharmaceutically acceptable diluents, carriers or excipients therefor.

An additional embodiment of the present invention is a process for producing a protein of Formula (I), which comprises:
(a) transforming a host cell with DNA that encodes the protein of Formula (I), said protein having an optional leader sequence;
(b) culturing the host cell and isolating the protein encoded in step (a); and, optionally,
(c) cleaving enzymatically the leader sequence to produce the protein of Formula (I).

### Detailed Description

For purposes of the present invention, as disclosed and claimed herein, the following terms and abbreviations are defined as follows:

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.

DNA -- Deoxyribonucleic acid.

EDTA -- an abbreviation for ethylenediamine tetraacetic acid.

Immunoreactive Protein(s) -- a term used to collectively describe antibodies, fragments of antibodies capable of binding antigens of a similar nature as the parent antibody molecule from which they are derived, and single chain polypeptide binding molecules as described in PCT Application No. PCT/US 87/02208, International Publication No. WO 88/01649.

mRNA -- messenger RNA.

Plasmid -- an extrachromosomal self-replicating genetic element.

PMSF -- an abbreviation for phenylmethylsulfonyl fluoride.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

RNA -- ribonucleic acid.

RP-HPLC -- an abbreviation for reversed-phase high performance liquid chromatography.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Tris -- an abbreviation for tris(hydroxymethyl)-aminomethane.

Treating -- describes the management and care of a patient for the purpose of combating the disease, condition, or disorder and includes the administration of a compound of present invention to prevent the onset of the symptoms or complications, alleviating the symptoms or complications, or eliminating the disease, condition, or disorder. Treating obesity therefor includes the inhibition of food intake, the inhibition of weight gain, and inducing weight loss in patients in need thereof.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

X-gal -- an abbreviation for 5-bromo-4-chloro-3-indolyl beta-D-galactoside.

The amino acid abbreviations are accepted by the United States Patent and Trademark Office as set forth in 37 C.F.R. § 1.822 (b) (2) (1993). One skilled in the art would recognize that certain amino acids are prone to rearrangement. For example, Asn may rearrange to aspartic acid and isoaspartate as described in I. Schön et al., Int. J. Peptide Protein Res. 14: 485-94 (1979) and references cited therein. These rearrangement derivatives are included within the scope of the present invention. Unless otherwise indicated the amino acids are in the L configuration.

As noted above the present invention provides a protein of the Formula (I). Preferred proteins are those of Formula (II): 
(SEQ ID NO: 2)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (II)
 wherein:
Asn at position 22 is optionally Gln or Asp;
Thr at position 27 is optionally Ala;
Gln at position 28 is optionally Glu or absent;
Met at position 54 is optionally Ala;
Met at position 68 is optionally Leu;
Asn at position 72 is optionally Glu, or Asp;
Ser at position 77 is optionally Ala;
Gly at position 118 is optionally Leu;
said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

Preferred proteins are of the Formula II, wherein: Trp at position 100 is Gln, Tyr, Phe, Ile, Val, or Leu; or Trp at position 138 is Gln, Tyr, Phe, Ile, Val, or Leu.

Other preferred proteins of the Formula III: 
(SEQ ID NO: 3)
5                   10                  15
Val Pro Ile Gln Lys Val Gln Asp Asp Thr Lys Thr Leu Ile Lys Thr
 
20
Ile Val Thr Arg Ile Asn Asp Ile Ser His Thr Gln Ser Val Ser Ser
25
30

35
Lys Gln Lys Val Thr Gly Leu Asp Phe Ile Pro Gly Leu His Pro Ile
40
45

50
Leu Thr Leu Ser Lys Met Asp Gln Thr Leu Ala Val Tyr Gln Gln Ile
55
60

65
Leu Thr Ser Met Pro Ser Arg Asn Val Ile Gln Ile Ser Asn Asp Leu
70
75
80

85
Glu Asn Leu Arg Asp Leu Leu His Val Leu Ala Phe Ser Lys Ser Cys
90
95

100
His Leu Pro Trp Ala Ser Gly Leu Glu Thr Leu Asp Ser Leu Gly Gly
105
110

115
Val Leu Glu Ala Ser Gly Tyr Ser Thr Glu Val Val Ala Leu Ser Arg
120
125

130
Leu Gln Gly Ser Leu Gln Asp Met Leu Trp Gln Leu Asp Leu Ser Pro
135
140

145
Gly Cys
(III)
 wherein:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val, or Leu;
Ala at position 101 is replaced with Ser, Asn, Gly, His, Pro, Thr or Val;
Ser at position 102 is replaced with Arg;
Gly at position 103 is replaced with Ala;
Glu at position 105 is replaced with Gln;
Thr at position 106 is replaced with Lys or Ser; Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

Most preferred proteins are those of Formula III, wherein:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val, or Leu;
Ala at position 101 is replaced with Ser, Asn, Gly, His, Pro, Thr or Val;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is Ala, Glu, Asp, Asn, Met, Ile, Phe, Tyr, Ser, Thr, Gly, Gln, Val or Leu.

Still more preferred proteins of the Formula III are those wherein:
His at position 97 is replaced with Ser or Pro;
Trp at position 100 is replaced with Ala, Gly, Gln, Val, Ile, or Leu;
Ala at position 101 is replaced with Thr; or
Trp at position 138 is Ala, Ile, Gly, Gln, Val or Leu.

Additional preferred proteins of the Formula III are those wherein:
His at position 97 is replaced with Ser or Pro;
Trp at position 100 is replaced with Ala, Gln or Leu;
Ala at position 101 is replaced with Thr; or
Trp at position 138 is Gln.

Additional preferred proteins of the present invention include proteins of SEQ ID NO: 3, wherein the amino acid residues at positions 97, 100, 101, 105, 106, 107, 108, and 111 are substituted as follows in Table 1:

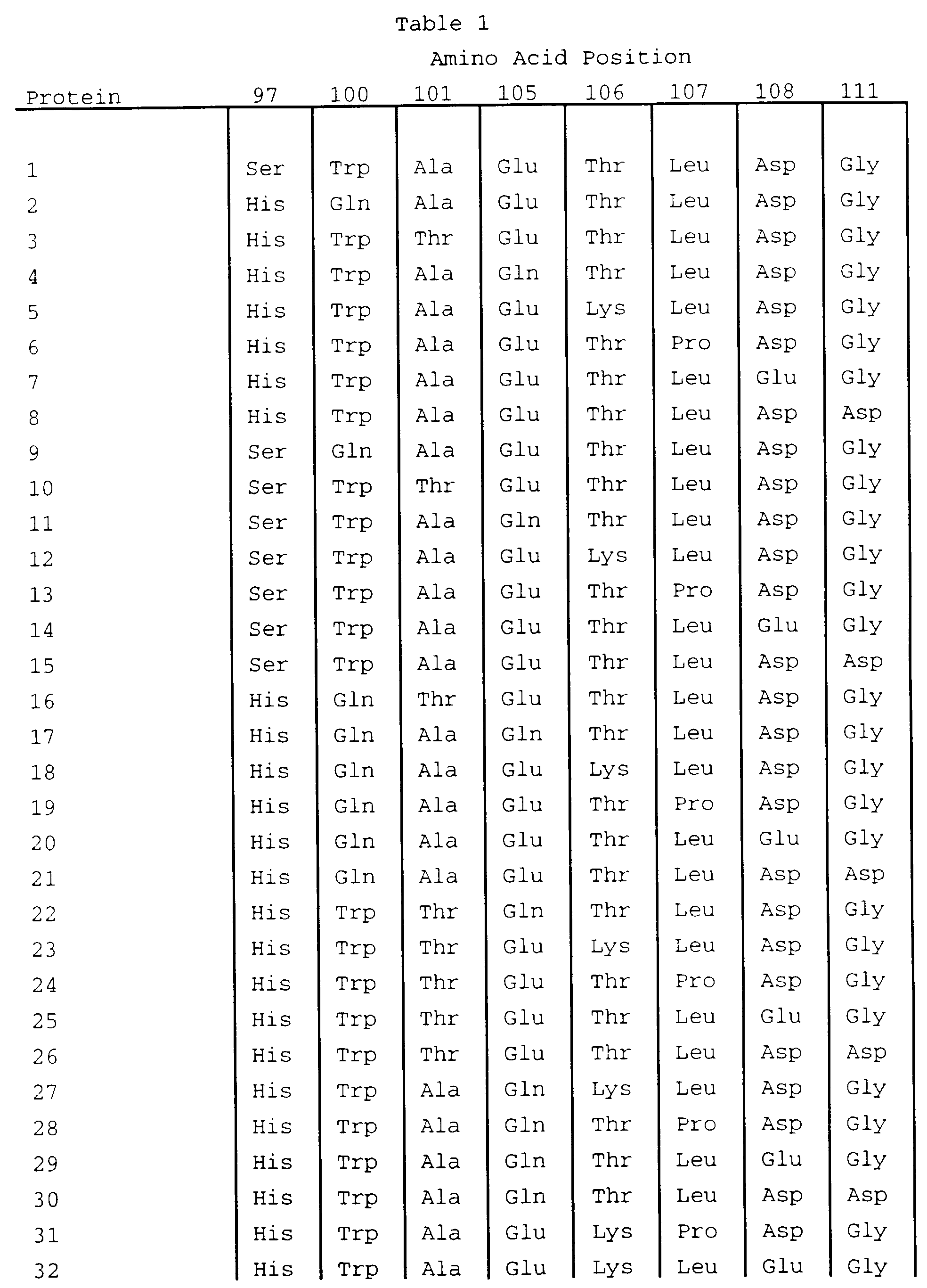

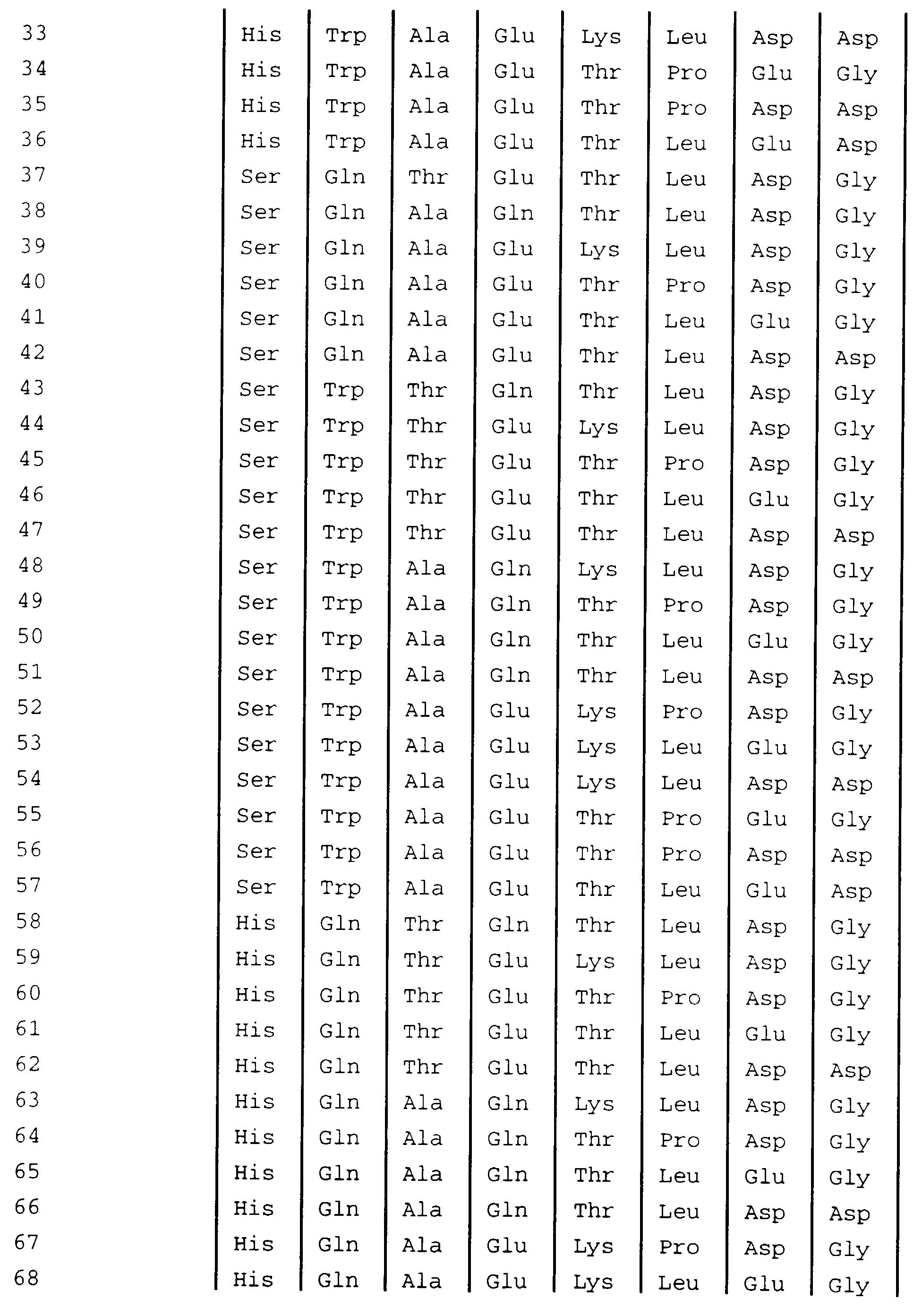

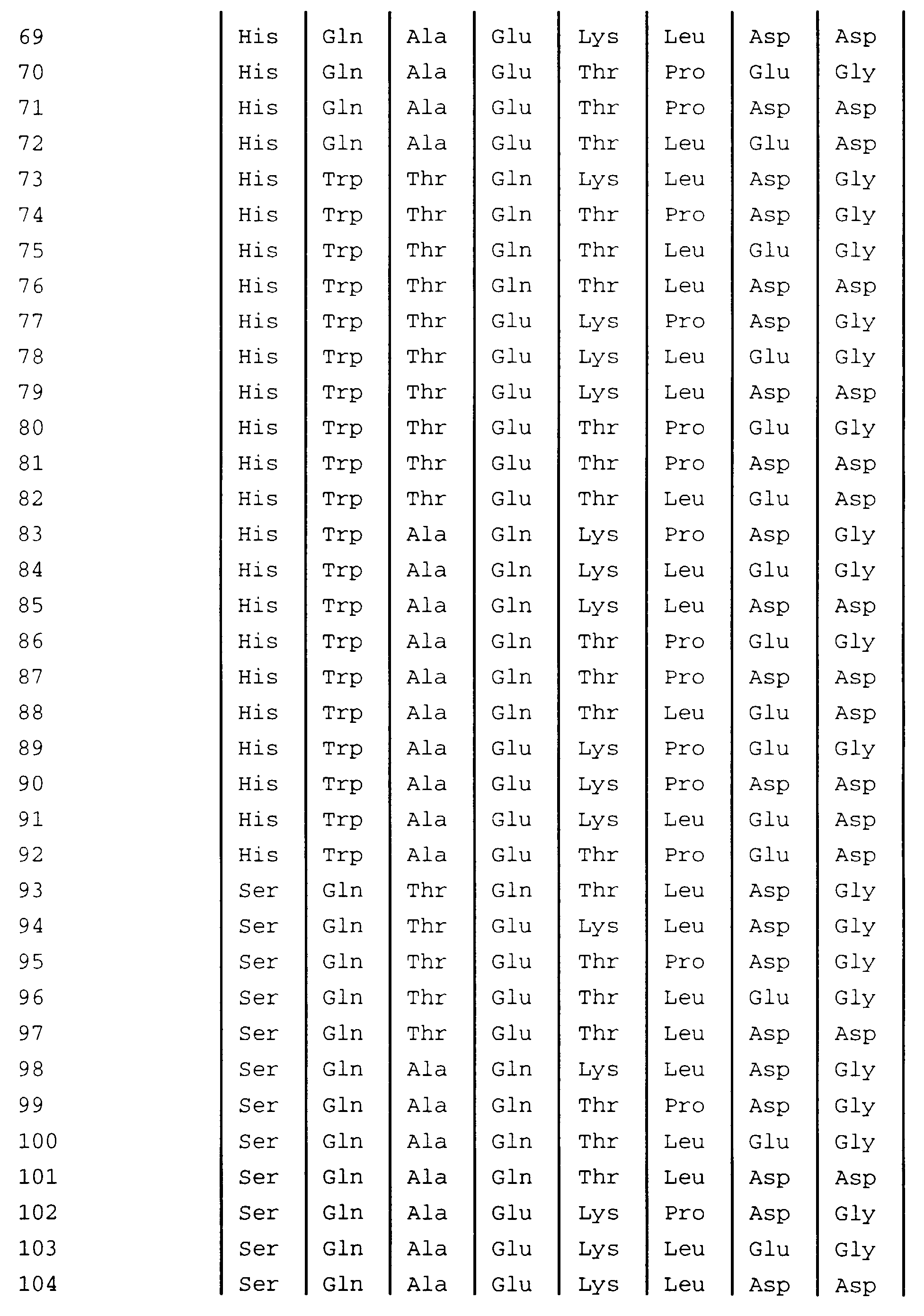

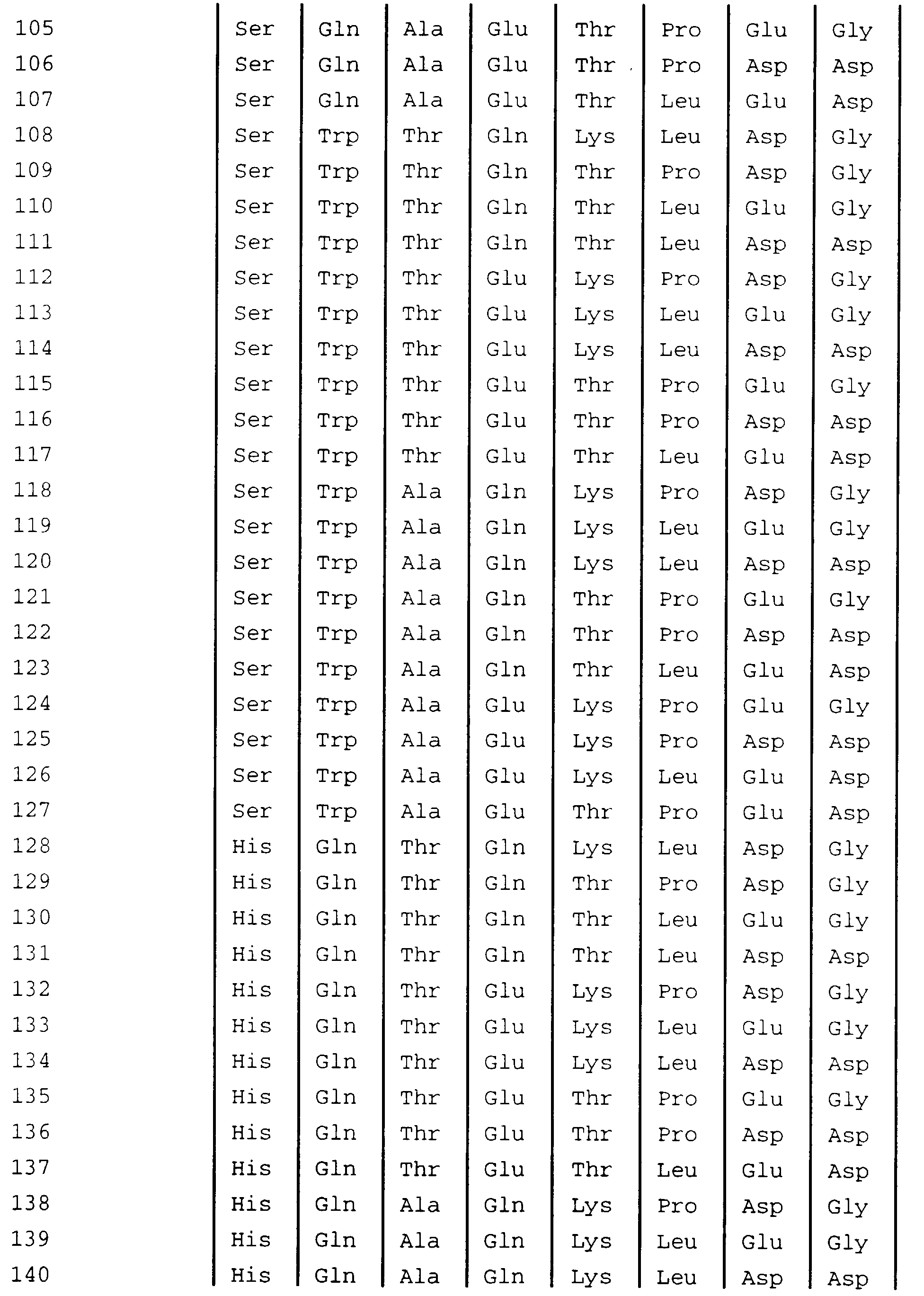

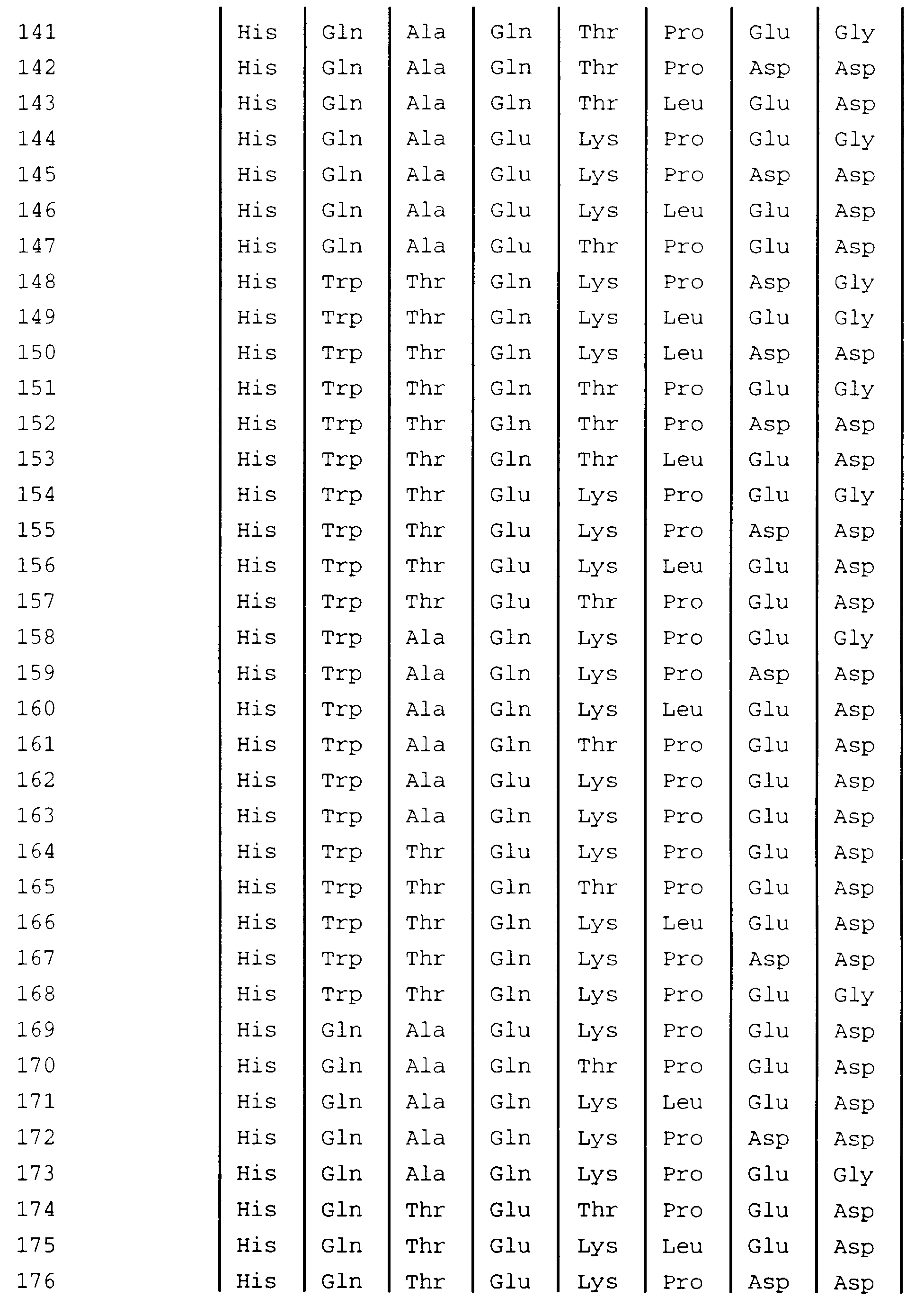

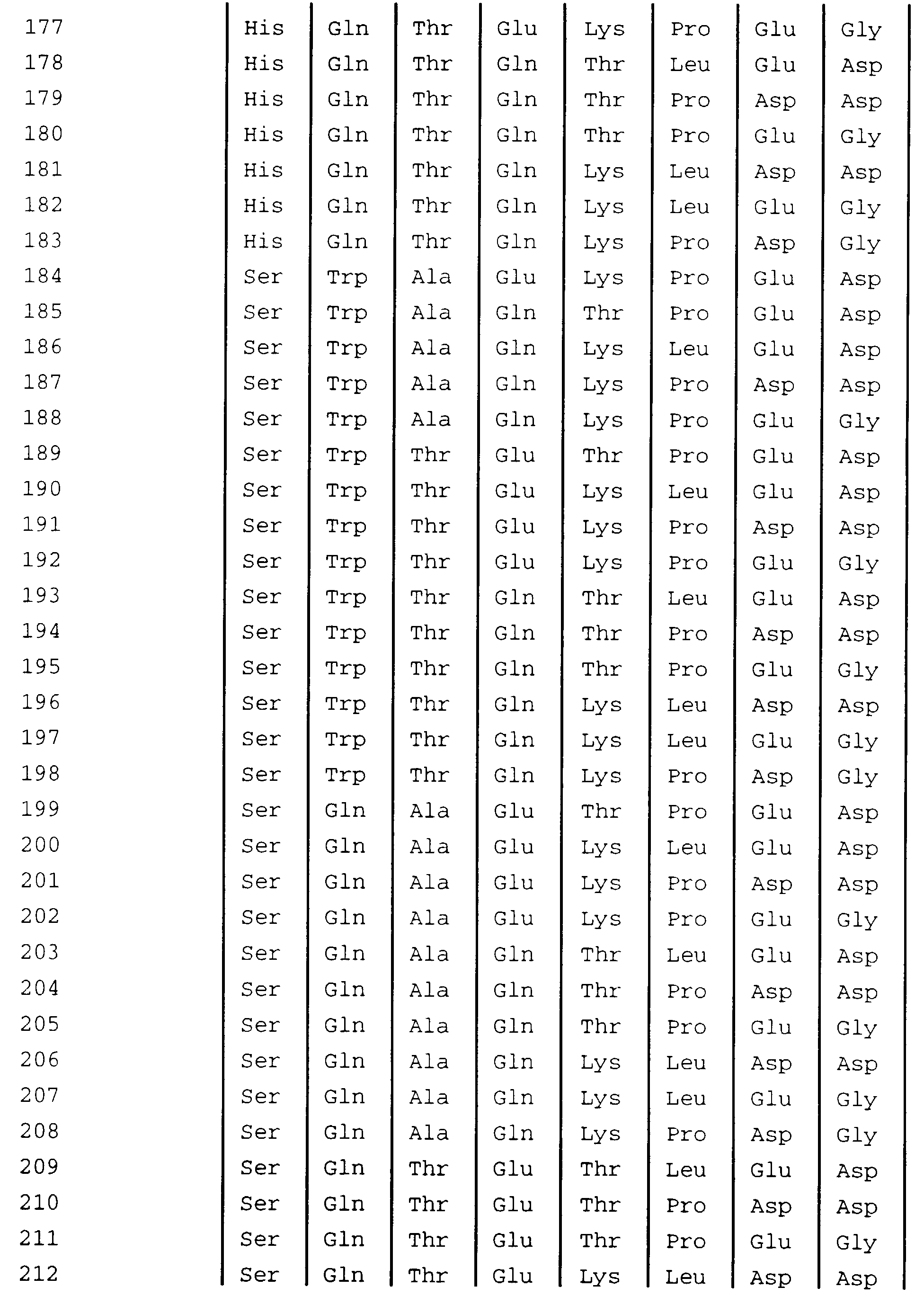

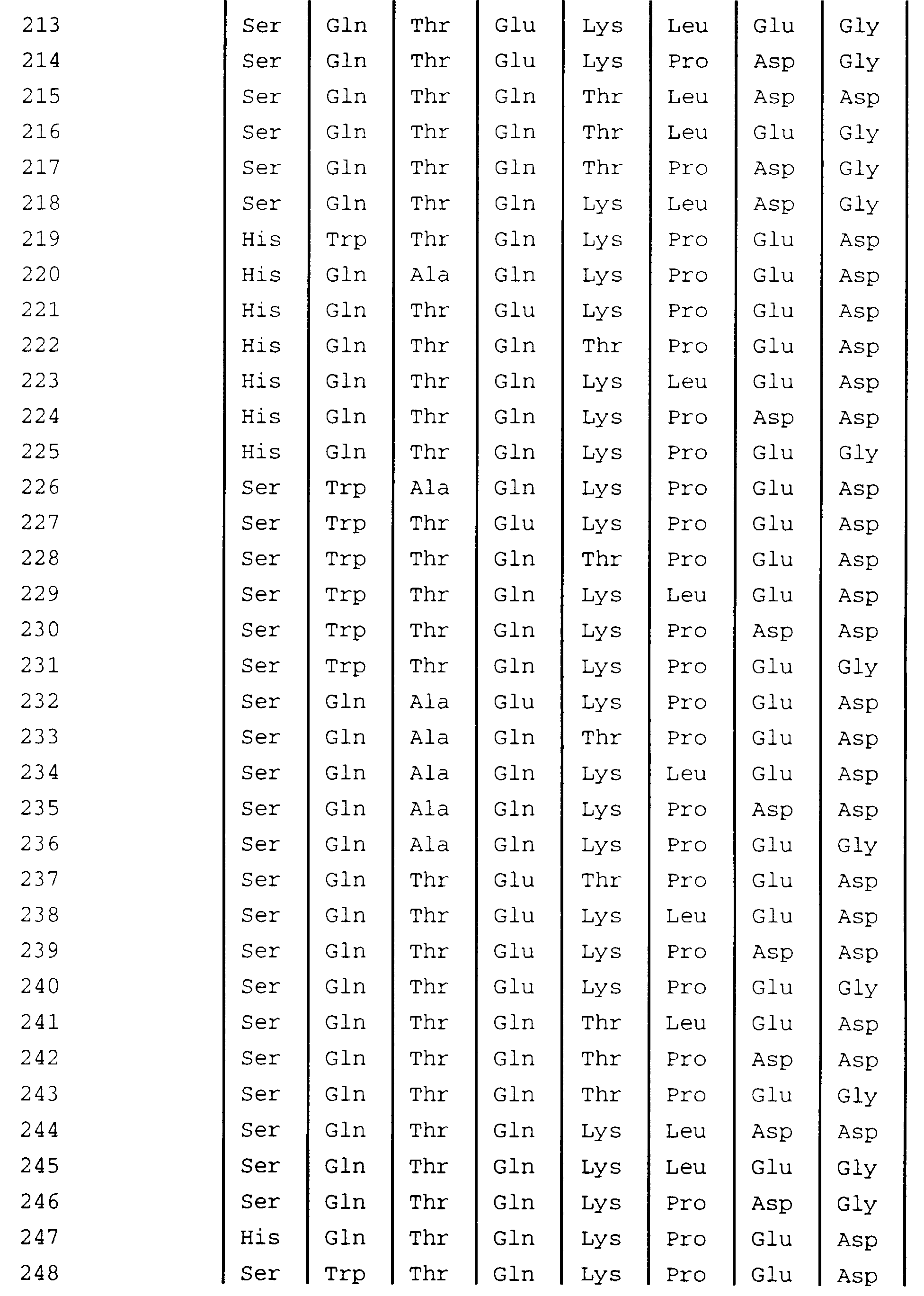

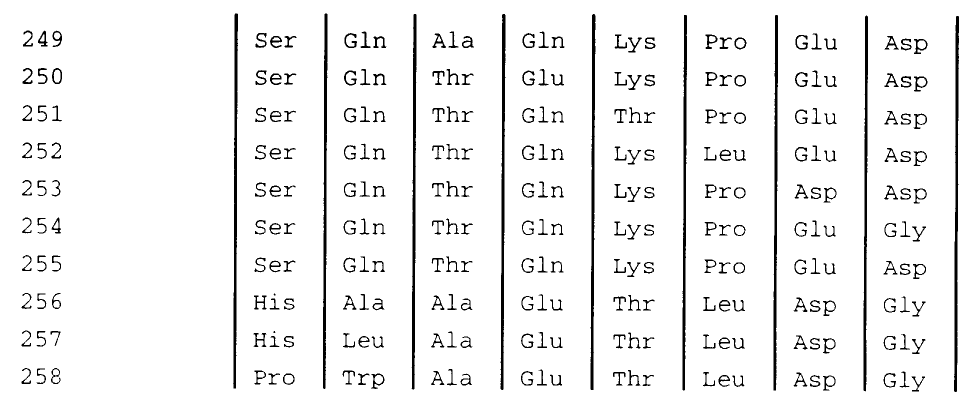

Most preferred species of Formula III and Table 1 include species of SEQ ID NO: 4-11:

(SEQ ID NO: 4)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys

(SEQ ID NO: 5)
5 10 15
Val Pro Ile Gln Lys Val Gln Asp Asp Thr Lys Thr Leu Ile Lys Thr

20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Gln Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys

(SEQ ID NO: 6)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

(SEQ ID NO: 7)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

(SEQ ID NO: 8)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

        130                 135                 140
Leu Gln Gly Ser Leu Gln Asp Met Leu Trp Gln Leu Asp Leu Ser Pro

145
Gly Cys

(SEQ ID NO: 9)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

(SEQ ID NO: 10)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
Ser Leu Pro Gln Thr Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

(SEQ ID NO: 11)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
Ser Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

The present invention provides biologically active proteins that provide effective treatment for obesity. Unexpectedly, the claimed proteins have improved properties due to specific substitutions to the human obesity protein. The claimed proteins are more stable than both the mouse and human obesity protein and, therefore, are superior therapeutic agents.

The claimed proteins ordinarily are prepared by recombinant techniques. Techniques for making substitutional mutations at predetermined sites in DNA having a known sequence are well known, for example M13 primer mutagenesis. The mutations that might be made in the DNA encoding the present anti-obesity proteins must not place the sequence out of reading frame and preferably will not create complementary regions that could produce secondary mRNA structure. See DeBoer et al., EP 75,444A (1983).

The compounds of the present invention may be produced either by recombinant DNA technology or well known chemical procedures, such as solution or solid-phase peptide synthesis, or semi-synthesis in solution beginning with protein fragments coupled through conventional solution methods.

### A. Solid Phase

The synthesis of the claimed proteins may proceed by solid phase peptide synthesis or by recombinant methods. The principles of solid phase chemical synthesis of polypeptides are well known in the art and may be found in general texts in the area such as Dugas, H. and Penney, C., Bioorganic Chemistry Springer-Verlag, New York, pgs. 54-92 (1981). For example, peptides may be synthesized by solid-phase methodology utilizing an PE-Applied Biosystems 433A peptide synthesizer (commercially available from Applied Biosystems, Foster City California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine and Methionine are coupled using preformed hydroxy benzotriazole esters. The following side chain protection may be used:
Arg, Tosyl
Asp, cyclohexyl or benzyl
Cys, 4-methylbenzyl
Glu, cyclohexyl
His, benzyloxymethyl
Lys, 2-chlorobenzyloxycarbonyl
Met, sulfoxide
Ser, Benzyl
Thr, Benzyl
Trp, formyl
Tyr, 4-bromo carbobenzoxy
Boc deprotection may be accomplished with trifluoroacetic acid (TFA) in methylene chloride. Formyl removal from Trp is accomplished by treatment of the peptidyl resin with 20% piperidine in dimethylformamide for 60 minutes at 4°C. Met(O) can be reduced by treatment of the peptidyl resin with TFA/dimethylsulfide/conHCl (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/dimethylsulfide. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography (Vydac) column in .1% TFA with a gradient of increasing acetonitrile concentration.

One skilled in the art recognizes that the solid phase synthesis could also be accomplished using the FMOC strategy and a TFA/scavenger cleavage mixture.

### B. Recombinant Synthesis

The claimed proteins may also be produced by recombinant methods. Recombinant methods are preferred if a high yield is desired. The basic steps in the recombinant production of protein include:
a) construction of a synthetic or semi-synthetic (or isolation from natural sources) DNA encoding the claimed protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.

### a. Gene Construction

Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the claimed proteins. In the preferred practice of the invention, synthesis is achieved by recombinant DNA technology.

Methodology of synthetic gene construction is well known in the art. For example, see Brown, et al. (1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404).

It may desirable in some applications to modify the coding sequence of the claimed protein so as to incorporate a convenient protease sensitive cleavage site, e.g., between the signal peptide and the structural protein facilitating the controlled excision of the signal peptide from the fusion protein construct.

The gene encoding the claimed protein may also be created by using polymerase chain reaction (PCR). The template can be a cDNA library (commercially available from CLONETECH or STRATAGENE) or mRNA isolated from human adipose tissue. Such methodologies are well known in the art Maniatis, et al. Molecular Cloning: A Laboratory Manual, Cold Spring Harbor Press, Cold Spring Harbor Laboratory, Cold Spring Harbor, New York (1989).

### b. Direct expression or Fusion protein

The claimed protein may be made either by direct expression or as fusion protein comprising the claimed protein followed by enzymatic or chemical cleavage. A variety of peptidases (e.g. trypsin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., Carter P., Site Specific Proteolysis of Fusion Proteins, Ch. 13 in Protein Purification: From Molecular Mechanisms to Large Scale Processes, American Chemical Soc., Washington, D.C. (1990).

### c. Vector Construction

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The choice of restriction sites are chosen so as to properly orient the coding sequence with control sequences to achieve proper in-frame reading and expression of the claimed protein.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The genes encoding the protein of the present invention can be inserted into the plasmid backbone on a NdeI/BamHI restriction fragment cassette.

### d. Procaryotic expression

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Prokaryotes also are used for expression. The aforementioned strains, as well as E. *c*oli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

### e. Eucaryotic expression

The protein may be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBβ (ATCC 77177). Of course, promoters from the host cell or related species also are useful herein.

Transcription of a DNA encoding the claimed protein by higher eukaryotes is increased by inserting an enhancer sequence into the vector. Enhancers are cis-acting elements of DNA, usually about 10-300 bp, that act on a promoter to increase its transcription. Enhancers are relatively orientation and position independent having been found 5' (Laimins, L. et al., PNAS 78:993 (1981)) and 3' (Lusky, M. L., et al., Mol. Cell Bio. 3:1108 (1983)) to the transcription unit, within an intron (Banerji, J. L. et al., cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH5a (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eukaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); Chinese hamster ovary cells CHO-DHFR⁻ (ATCC CRL-9096), mouse Sertoli cells (TM4, ATCC CRL-1715, Biol. Reprod. 23:243-250 (1980)); African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

### f. Yeast expression

In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hI1beta ATCC 67024), also are advantageously used with yeast promoters.

The following examples are presented to further illustrate the preparation of the claimed proteins. The scope of the present invention is not to be construed as merely consisting of the following examples.

### Example 1

### Vector Construction

A gene of SEQ ID NO:12 is assembled from a ∼220 base pair and a ∼240 base pair segment which are derived from chemically synthesized oligonucleotides. 
(SEQ ID NO: 12)
1 CATATGAGGG TACCTATCCA AAAAGTACAA GATGACACCA AAACACTGAT
 
51 AAAGACAATA GTCACAAGGA TAAATGATAT CTCACACACA CAGTCAGTCT
101 CATCTAAACA GAAAGTCACA GGCTTGGACT TCATACCTGG GCTGCACCCC
151 ATACTGACAT TGTCTAAAAT GGACCAGACA CTGGCAGTCT ATCAACAGAT
201 CTTAACAAGT ATGCCTTCTA GAAACGTGAT ACAAATATCT AACGACCTGG
251 AGAACCTGCG GGATCTGCTG CACGTGCTGG CCTTCTCTAA AAGTTGCCAC
301 TTGCCATGGG CCAGTGGCCT GGAGACATTG GACAGTCTGG GGGGAGTCCT
351 GGAAGCCTCA GGCTATTCTA CAGAGGTGGT GGCCCTGAGC AGGCTGCAGG
401 GGTCTCTGCA AGACATGCTG TGGCAGCTGG ACCTGAGCCC CGGGTGCTAA
451 TAGGATCC
 The 220 base pair segment extends from the NdeI site to the XbaI site at position 220 within the coding region and is assembled from 7 overlapping oligonucleotides which range in length from between 34 and 83 bases. The 240 base pair segment which extends from the XbaI to the BamHI site is also assembled from 7 overlapping oligonucleotides which range in length from between 57 and 92 bases.

To assemble these fragments, the respective 7 oligonucleotides are mixed in equimolar amounts, usually at concentrations of about 1-2 picomoles per microliters. Prior to assembly, all but the oligonucleotides at the 5" -ends of the segment are phosphorylated in standard kinase buffer with T4 DNA kinase using the conditions specified by the supplier of the reagents. The mixtures are heated to 95 degrees and allowed to cool slowly to room temperature over a period of 1-2 hours to ensure proper annealing of the oligonucleotides. The oligonucleotides are then ligated to each other and into an appropriated cloning vector such as pUC18 or pUC 19 using T4 DNA ligase. The buffers and conditions are those recommended by the supplier of the enzyme. The vector for the 220 base pair fragment is digested with NdeI and XbaI, whereas the vector for the 240 base pair fragment is digested with XbaI and BamHI prior to use. The ligation mixes are used to transform E. coli DH10B cells (commercially available from Gibco/BRL) and the transformed cells are plated on tryptone-yeast (TY) plates containing 100 µg/ml of ampicillin, X-gal and IPTG. Colonies which grow up overnight are grown in liquid TY medium with 100 µg/ml of ampicillin and are used for plasmid isolation and DNA sequence analysis. Plasmids with the correct sequence are kept for the assembly of the complete gene. This is accomplished by gel-purification of the 220 base-pair and the 240 base-pair fragments and ligation of these two fragments into an expression vector such as pRB182 from which the coding sequence for A-C-B proinsulin is deleted and is digested with NdeI and BamHI prior to use.

### Example 2

The plasmid containing the DNA sequence encoding the desired protein, is digested with PmlI and Bsu36I. The recognition sequences for these enzymes lie within the coding region for the protein at nucleotide positions 275 and 360 respectively. The cloning vector does not contain these recognition sequences. Consequently, only two fragments are seen following restriction enzyme digestion with PmlI and Bsu36I, one corresponding to the vector fragment, the other corresponding to the -85 base pair fragment liberated from within the protein coding sequence. This sequence can be replaced by any DNA sequence encoding the amino acid substitutions listed in Table 1. These DNA sequences are synthesized chemically as two oligonucleotides with complementary bases and ends that are compatible with the ends generated by digestion with PmlI and Bsu36I. The chemically synthesized oligonucleotides are mixed in equimolar amounts (1-10 picomoles/microliter), heated to 95 degrees and allow to anneal by slowly decreasing the temperature to 20-25 degrees. The annealed oligonucleotides are used in a standard ligation reaction. Ligation products are tranformed and analysed as described in Example 1.

### Example 3

A DNA sequence encoding a protein represented by Protein 255 in Table 1 with a Met Arg leader sequence was obtained using the plasmid and procedures described in Example 2. The plasmid was digested with PmlI and Bsu36I. A synthetic DNA fragment of the sequence 5"-SEQ ID NO:13: 
(SEQ ID NO: 13)
GTGCTGGCCTTCTCTAAAAGTTGCAGCTTGCCACAGACCAGTGGCCTGCAGAAACCGGAAA
GTCTGGACGGAGTCCTGGAAGCC
 annealed with the sequence 5'-SEQ ID NO:14: 
(SEQ ID NO: 14)
TGAGGCTTCCAGGACTCCGTCCAGACTTTCCGGTTTCTGCAGGCCACTGGTCTGTGGCAAG
CTGCAACTTTTAGAGAAGGCCAGCAC
 was inserted between the PmlI and the Bsu36I sites. Following ligation, transformation and plasmid isolation, the sequence of the synthetic fragment was verified by DNA sequence analysis.

### Example 4

A DNA sequence encoding SEQ ID NO: 4 with a Met Arg leader sequence was obtained using the plasmid and procedures described in Example 2. The plasmid was digested with PmlI and Bsu36I. A synthetic DNA fragment of the sequence 5"-SEQ ID NO:15 
(SEQ ID NO: 15)
GTGCTGGCCTTCTCTAAAAGTTGCCACTTGCCAGCTGCCAGTGGCCTGGAGACATTGGACA
GTCTGGGGGGAGTCCTGGAAGCC
 annealed with the sequence 5'-SEQ ID NO:16: 
(SEQ ID NO: 16)
TGAGGCTTCCAGGACTCCCCCCAGACTGTCCAATGTCTCCAGGCCACTGGCAGCTGGCAAG
TGGCAACTTTTAGAGAAGGCCAGCAC
 was inserted between the PmlI and the Bsu36I sites. Following ligation, transformation and plasmid isolation, the sequence of the synthetic fragment was verified by DNA sequence analysis.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins which are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be dissolved to provide further purification and isolation of the desired protein product. Id. A variety of techniques using strongly denaturing solutions such as guanidinium-HCl and/or weakly denaturing solutions such as urea are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

### Example 5

The protein of Example 3 with a Met Arg leader sequence was expressed in E.coli, isolated and folded either by dilution into PBS or by dilution into 8M urea (both containing 5 mM cysteine) and exhaustive dialysis against PBS. Little to no aggregation of protein was seen in either of these procedures. Following final purification of the proteins by size exclusion chromatography the proteins were concentrated to 3-3.5 mg/mL in PBS. Virtually no aggregation of the protein was noted in contrast to the native human protein for which substantial aggregation is noted upon concentration.

Analysis of the proteins by reverse phase HPLC indicated that the human Ob protein eluted at approximately 56.6 % acetonitrile, the mouse protein at 55.8 %, and the titled protein with a Met Arg leader sequence at 53.7 %. Thus, unexpectedly the human with the mouse insert appears to have higher hydrophilicity than either the human or mouse molecules.

### Example 6

The protein of SEQ ID NO: 4 with a Met Arg leader sequence was expressed in E.coli. Granules were isolated and solubilized in 8M urea with 5 mM cysteine. The protein was purified by anion exchange chromatography and folded by dilution into 8M urea (containing 5 mM cysteine) and exhaustive dialysis against PBS by techniques. The pH of the protein solution was reduced to about 2.8. The Met Arg leader sequence was cleaved by the addition of 6-10 milliunits dDAP per mg of protein. The conversion reaction was allowed to proceed for 2-8 hours at room temperature. The progress of the reaction was monitored by high performance reversed phase chromatography. The reaction was terminated by adjusting the pH to 8 with NaOH. The des(Met-Arg) protein was further purified by cation exchange chromatography in the presence of 7-8 M urea and size exclusion chromatography into PBS. Following final purification of the proteins by size exclusion chromatography the proteins were concentrated to 3-3.5 mg/mL in PBS. Virtually no aggregation of the protein was noted.

Preferably, the present proteins are expressed with a leader sequence. Operable leader sequences are known to one of ordinary skill in the art; however, preferably the leader sequence is Met-R₁-, wherein R₁ is any amino acid except Pro, so that the expressed proteins may be readily converted to the claimed protein with Cathepsin C. Preferably, R₁ is Arg, Asp, or Tyr; and most preferably, the proteins are expressed with a Met-Arg leader sequence. Interestingly, the leader sequence does not significantly affect stability or activity of the active protein. However, the leader sequence is preferably cleaved from the protein. Thus, the proteins of the Formula: Met-R₁-SEQ ID NO:1 are useful as biological agents and, preferably, as an intermediate.

The purification of the claimed proteins is by techniques known in the art and includes reverse phase chromatography, affinity chromatography, ion exchange and size exclusion chromatography.

The claimed proteins contain two cysteine residues. Thus, a di-sulfide bond may be formed to stabilize the protein. The present invention includes proteins of the Formula (I) or (II) wherein the Cys at position 96 is crosslinked to Cys at position 146 as well as those proteins without such di-sulfide bonds. In addition the proteins of the present invention may exist, particularly when formulated, as dimers, trimers, tetramers, and other multimers. Such multimers are included within the scope of the present invention.

The present invention provides a method for treating obesity. The method comprises administering to the organism an effective amount of anti-obesity protein in a dose between about 1 and 1000 µg/kg. A preferred dose is from about 10 to 100 µg/kg of active compound. A typical daily dose for an adult human is from about 0.5 to 100 mg. In practicing this method, compounds of the Formula (I) can be administered in a single daily dose or in multiple doses per day. The treatment regime may require administration over extended periods of time. The amount per administered dose or the total amount administered will be determined by the physician and depend on such factors as the nature and severity of the disease, the age and general health of the patient and the tolerance of the patient to the compound.

The instant invention further provides pharmaceutical formulations comprising compounds of the present invention. The proteins, preferably in the form of a pharmaceutically acceptable salt, can be formulated for parenteral administration for the therapeutic or prophylactic treatment of obesity. For example, compounds of the Formula (I) can be admixed with conventional pharmaceutical carriers and excipients. The compositions comprising claimed proteins contain from about 0.1 to 90% by weight of the active protein, preferably in a soluble form, and more generally from about 10 to 30%. Furthermore, the present proteins may be administered alone or in combination with other anti-obesity agents or agents useful in treating diabetes.

For intravenous (iv) use, the protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of a protein of the Formula (I) or (II), for example the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

The ability of the present compounds to treat obesity is demonstrated *in vivo* as follows:

### Biological Testing

Parabiotic experiments suggest that a protein is released by peripheral adipose tissue and that the protein is able to control body weight gain in normal, as well as obese mice. Therefore, the most closely related biological test is to inject the test article by any of several routes of administration (e.g. i.v., s.c., i.p., or by minipump or cannula) and then to monitor food and water consumption, body weight gain, plasma chemistry or hormones (glucose, insulin, ACTH, corticosterone, GH, T4) over various time periods.

Suitable test animals include normal mice (ICR, etc.) and obese mice *(ob/ob,* Avy/a, KK-Ay, tubby, fat). The *ob/ob* mouse model of obesity and diabetes is generally accepted in the art as being indicative of the obesity condition. Controls for non-specific effects for these injections are done using vehicle with or without the active agent of similar composition in the same animal monitoring the same parameters or the active agent itself in animals that are thought to lack the receptor (db/db mice, fa/fa or cp/cp rats). Proteins demonstrating activity in these models will demonstrate similar activity in other mammals, particularly humans.

Since the target tissue is expected to be the hypothalamus where food intake and lipogenic state are regulated, a similar model is to inject the test article directly into the brain (e.g. i.c.v. injection via lateral or third ventricles, or directly into specific hypothalamic nuclei (e.g. arcuate, paraventricular, perifornical nuclei). The same parameters as above could be measured, or the release of neurotransmitters that are known to regulate feeding or metabolism could be monitored (e.g. NPY, galanin, norepinephrine, dopamine, β-endorphin release).

Representative proteins outlined in Table 2 were prepared in accordance with the teachings and examples provided herein. The description of the protein in Table 2, and in subsequent Table 3, designates the substituted amino acids of SEQ ID NO: 3 as provided in Formula I. For example, Ala(100) designates a protein of SEQ ID NO: 3 wherein Trp at position 100 is Ala. The designation Met Arg - indicates that the protein was prepared and tested with the Met Arg leader sequence attached. Amino acid sequences of the proteins of Table 2 and 3 were confirmed by mass spectroscopy and/or amino acid analysis. The ability of the present proteins to treat obesity in a *OB/OB* mouse is also presented in Table 2.

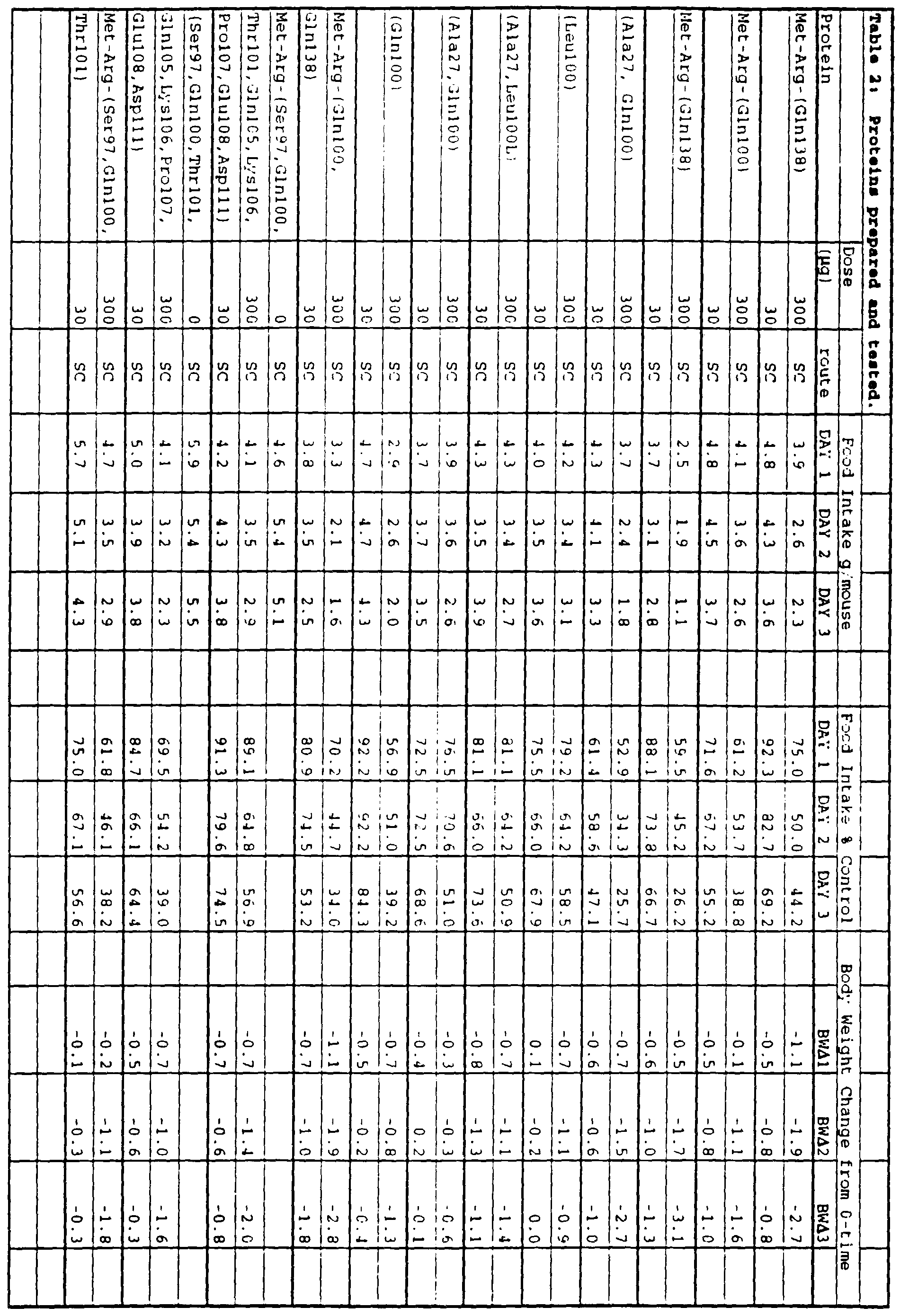

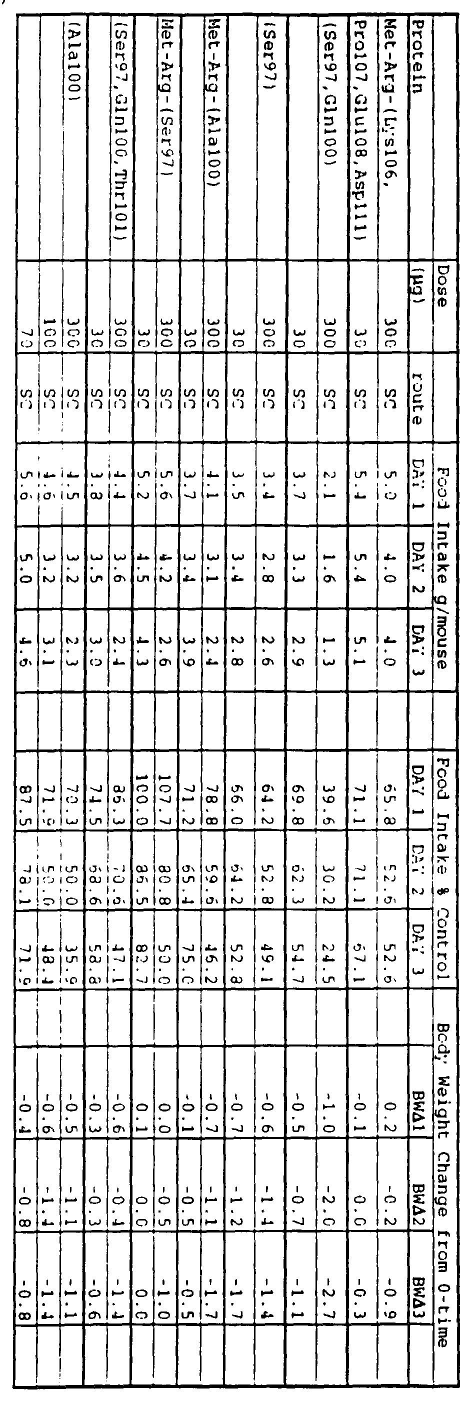

Similar studies are accomplished *in vitro* using isolated hypothalamic tissue in a perifusion or tissue bath system. In this situation, the release of neurotransmitters or electrophysiological changes is monitored.

The physical and chemical properties of the present compounds is demonstrated as follows.

### Shake Test

The starting solutions contain purified Ob protein in phosphate-buffered saline (Gibco BRL, Dulbecco's PBS without calcium phosphate or magnesium phosphate, from Life Technologies, Inc., Grand Island, NY). The protein concentrations are generally determined by their absorbence at 280 nm. However, an alternative method is employed for Ob proteins with a theoretical absorbance value at 280 nm of 0.5 or less for a 1 mg/mL solution in a 1-cm cuvette. The total integrated peak areas are determined from a 25 µL sample injected onto an analytical size-exclusion chromatography (SEC) column (Superdex-75, Pharmacia), which is run at ambient temperature in PBS and monitored at 214 nm. This peak area is then compared to the total SEC peak area of an Ob protein whose concentration was first determined by its absorbance at 280 nm. From these analyses, a dilution is made with PBS to give each Ob protein a final concentration of about 1.6 mg/mL. Aliquots of these solutions are adjusted to pH 5.0, pH 6.0, pH 7.0 and pH 8.0 using minute quantities of dilute acetic acid or dilute NaOH. These pH-adjusted solutions are then quantitated by the UV absorbence or SEC techniques.

The Ob protein solutions are then added to 2-mL glass autosampler vials (Varian Instrument Group, Sunnyvale, CA) each containing 15 Teflon balls one-eighth inch in diameter (Curtin Matheson Scientific, Florence, KY). Air bubbles are removed from the solutions in the vials with gentle shaking. The vials are slightly overfilled at the top and then closed with the Teflon-coated seal and screw cap. A separate vial is used for each shake test time period that is to be evaluated.

The test vials are placed in a rotation device in an incubator set precisely at 40°C. The vials are rotated end-over-end at a rate of 30 revolutions per minute, allowing the Teflon beads to move gently from the top of the vial to the bottom while remaining completely in the solution.

After pre-determined time periods, the contents of the vials are removed and centrifuged 5 minutes at ambient temperature (Fisher Scientific Model 235C Centrifuge). The protein concentrations in the clear supernatants are determined again by the UV absorbence or SEC techniques. The percent of Ob protein remaining in solution is calculated from the Ob concentrations in the pH-adjusted starting solutions and in the supernatants after the shake test.

The chemical and physical stability of the present compounds is demonstrated in Table 3. The description of the protein in Table 3 designates the substituted amino acids of SEQ ID NO: 3 as provided in Formula (I). For example, Ala(100) designates a protein of SEQ ID NO: 3 wherein Trp at position 100 is replaced with Ala. For reference the human Ob protein and the mouse ob protein are also presented.

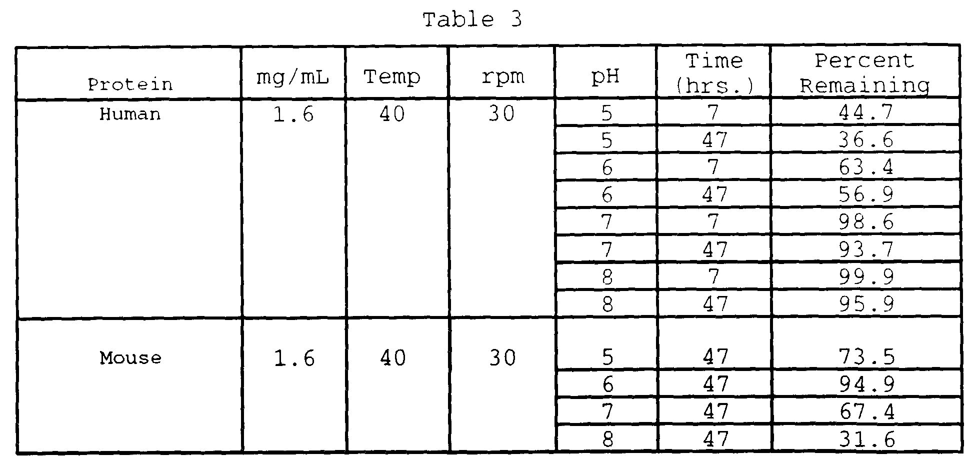

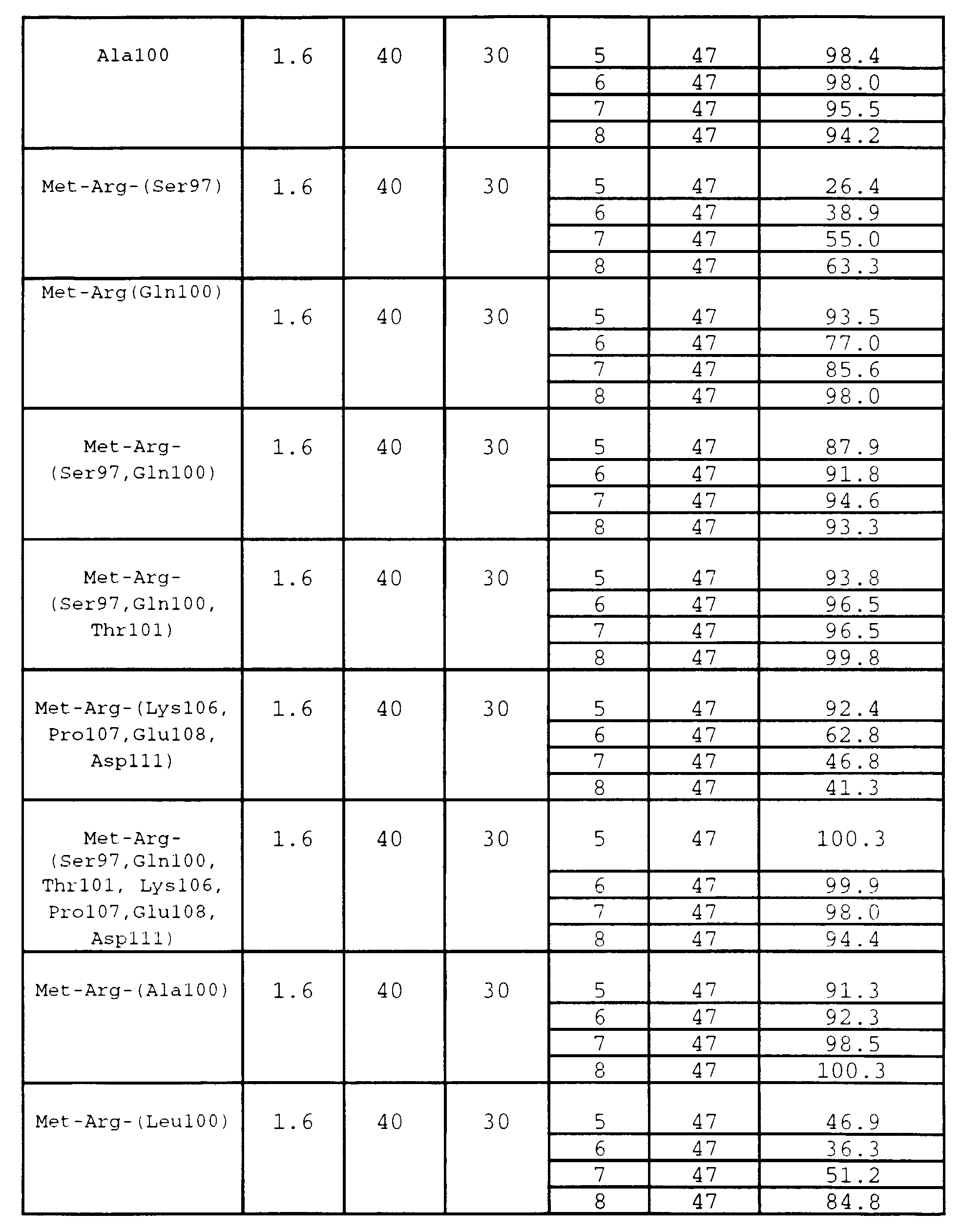

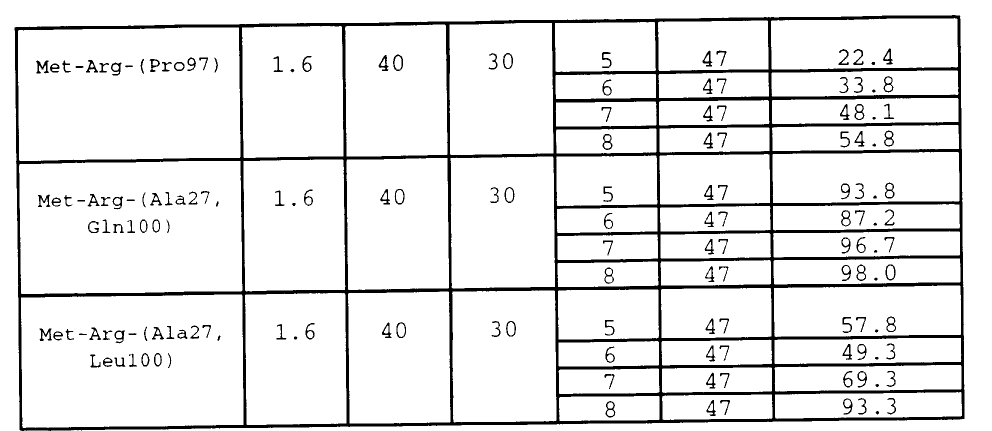

The compounds are active in at least one of the above biological tests and are anti-obesity agents. As such, they are useful in treating obesity and those disorders implicated by obesity. However, the proteins are not only useful as therapeutic agents; one skilled in the art recognizes that the proteins are useful in the production of antibodies for diagnostic use and, as proteins, are useful as feed additives for animals. Furthermore, the compounds are useful for controlling weight for cosmetic purposes in mammals. A cosmetic purpose seeks to control the weight of a mammal to improve bodily appearance. The mammal is not necessarily obese. Such cosmetic use forms part of the present invention.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A protein of the Formula (I): 
(SEQ ID NO: 1)

Val Pro Ile Xaa Lys Val Xaa Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Xaa Lys Val Thr Gly Leu Asp Phe Ile Pro Gly Leu His Pro Ile
        35                  40                  45

Leu Thr Leu Ser Lys Xaa Asp Xaa Thr Leu Ala Val Tyr Xaa Xaa Ile
    50                  55                  60

Leu Thr Ser Xaa Pro Ser Arg Xaa Val Ile Xaa Ile Xaa Xaa Asp Leu
65                  70                  75                  80

Glu Xaa Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Xaa Tyr Ser Thr Glu Val Val Ala Leu Ser Arg
        115                 120                 125

Leu Xaa Gly Ser Leu Xaa Asp Xaa Leu Trp Xaa Leu Asp Leu Ser Pro
    130                 135                 140

Gly Cys                                                     (I)
145
 wherein:
Xaa at position 4 is Gln or Glu;
Xaa at position 7 is Gln or Glu;
Xaa at position 22 is Asn, Asp or Glu;
Xaa at position 27 is Thr or Ala;
Xaa at position 28 is Gln, Glu, or absent;
Xaa at position 34 is Gln or Glu;
Xaa at position 54 is Met, methionine sulfoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Asn, Asp or Glu;
Xaa at position 75 is Gln or Glu;
Xaa at position 77 is Ser or Ala;
Xaa at position 78 is Gln, Asn, or Asp;
Xaa at position 82 is Gln, Asn, or Asp;
Xaa at position 118 is Gly or Leu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 139 is Gln or Glu;
said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

2. A protein of Claim 1 having at least one substitution selected from the group consisting of:
His at position 97 is replaced with Gln, Asn, Ala, Gly, Ser or Pro;
Trp at position 100 is replaced with Ala, Glu, Asp, Asn, Met, Ile, Phe, Tyr, Ser, Thr, Gly, Gln or Leu;
Ala at position 101 is replaced with Ser, Asn, Gly, His, Pro, Thr or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu; or
Gly at position 111 is replaced with Asp;
or a pharmaceutically acceptable salt thereof.

3. A protein of the Formula (II): 
(SEQ ID NO: 2)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (II)
 wherein:
Asn at position 22 is optionally Gln or Asp;
Thr at position 27 is optionally Ala;
Gln at position 28 is optionally Glu or absent;
Met at position 54 is optionally Ala;
Met at position 68 is optionally Leu;
Asn at position 72 is optionally Glu, or Asp;
Ser at position 77 is optionally Ala;
Gly at position 118 is optionally Leu;
said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

4. A protein of Claim 3, wherein:
Trp at position 100 is Gln, Tyr, Phe, Ile, Val, or Leu; or
Trp at position 138 is Gln, Tyr, Phe, Ile, Val, or Leu.

5. A protein of the Formula III: 
(SEQ ID NO: 3)
                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser
 
35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (III)
 wherein:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val, or Leu;
Ala at position 101 is replaced with Ser, Asn, Gly, His, Pro, Thr or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

6. A protein of Claim 5, wherein:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val, or Leu;
Ala at position 101 is replaced with Ser, Asn, Gly, His, Pro, Thr or Val;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is Ala, Glu, Asp, Asn, Met, Ile, Phe, Tyr, Ser, Thr, Gly, Gln, Val or Leu.

7. A protein of Claim 6, wherein:
His at position 97 is replaced with Ser or Pro;
Trp at position 100 is replaced with Ala, Gly, Gln, Val, Ile, or Leu;
Ala at position 101 is replaced with Thr; or
Trp at position 138 is Ala, Ile, Gly, Gln, Val or Leu.

8. A protein of any one of Claim 1 through 7, wherein the Cys at position 96 is di-sulfide bonded to the Cys at position 146.

9. A protein of SEQ ID NO: 4: 
(SEQ ID NO: 4)
5 10 15
Val Pro Ile Gln Lys Val Gln Asp Asp Thr Lys Thr Leu Ile Lys Thr
20 25 30
Ile Val Thr Arg Ile Asn Asp Ile Ser His Thr Gln Ser Val Ser Ser
35 40 45
Lys Gln Lys Val Thr Gly Leu Asp Phe Ile Pro Gly Leu His Pro Ile
50 55 60
Leu Thr Leu Ser Lys Met Asp Gln Thr Leu Ala Val Tyr Gln Gln Ile
65 70 75 80
Leu Thr Ser Met Pro Ser Arg Asn Val Ile Gln Ile Ser Asn Asp Leu
85 90 95
Glu Asn Leu Arg Asp Leu Leu His Val Leu Ala Phe Ser Lys Ser Cys
 
                100                     105                     110
His Leu Pro Ala Ala Ser Gly Leu Glu Thr Leu Asp Ser Leu Gly Gly

        115                     120                     125
Val Leu Glu Ala Ser Gly Tyr Ser Thr Glu Val Val Ala Leu Ser Arg

    130                     135                     140
Leu Gln Gly Ser Leu Gln Asp Met Leu Trp Gln Leu Asp Leu Ser Pro

145
Gly Cys
 wherein the Cys at position 96 is di-sulfide bonded to the Cys at position 146;
or a pharmaceutically acceptable salt thereof.

10. A protein of SEQ ID NO: 5: 
(SEQ ID NO: 5)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 wherein the Cys at position 96 is di-sulfide bonded to the Cys at position 146;
or a pharmaceutically acceptable salt thereof.

11. A protein of SEQ ID NO: 6: 
(SEQ ID NO: 6)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 wherein the Cys at position 96 is di-sulfide bonded to the Cys at position 146;
or a pharmaceutically acceptable salt thereof.

12. A protein of SEQ ID NO: 7: 
(SEQ ID NO: 7)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys
 
100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 wherein the Cys at position 96 is di-sulfide bonded to the Cys at position 146;
or a pharmaceutically acceptable salt thereof.

13. A protein of SEQ ID NO: 8: 
(SEQ ID NO: 8)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 wherein the Cys at position 96 is di-sulfide bonded to the Cys at position 146;
or a pharmaceutically acceptable salt thereof.

14. A protein of the formula: 
Met-R1-Val Pro Ile Xaa Lys Val Xaa Asp Asp Thr Lys Thr Leu Ile
1               5                   10

Lys Thr Ile Val Thr Arg Ile Xaa Asp Ile Ser His Xaa Xaa Ser Val
15                  20                  25                  30

Ser Ser Lys Xaa Lys Val Thr Gly Leu Asp Phe Ile Pro Gly Leu His
            35                  40                  45

Pro Ile Leu Thr Leu Ser Lys Xaa Asp Xaa Thr Leu Ala Val Tyr Xaa
        50                  55                  60

Xaa Ile Leu Thr Ser Xaa Pro Ser Arg Xaa Val Ile Xaa Ile Xaa Xaa
    65                  70                  75

Asp Leu Glu Xaa Leu Arg Asp Leu Leu His Val Leu Ala Phe Ser Lys
    80                  85                  90

Ser Cys His Leu Pro Trp Ala Ser Gly Leu Glu Thr Leu Asp Ser Leu
95                  100                 105                 110

Gly Gly Val Leu Glu Ala Ser Xaa Tyr Ser Thr Glu Val Val Ala Leu
            115                 120                 125

Ser Arg Leu Xaa Gly Ser Leu Xaa Asp Xaa Leu Trp Xaa Leu Asp Leu
        130                 135                 140

Ser Pro Gly Cys
    145
 wherein:
R¹ is any amino acid except Pro;
Xaa at position 4 is Gln or Glu;
Xaa at position 7 is Gln or Glu;
Xaa at position 22 is Asn, Asp or Glu;
Xaa at position 27 is Thr or Ala;
Xaa at position 28 is Gln, Glu, or absent;
Xaa at position 34 is Gln or Glu;
Xaa at position 54 is Met, methionine sulfoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Asn, Asp or Glu;
Xaa at position 75 is Gln or Glu;
Xaa at position 77 is Ser or Ala;
Xaa at position 78 is Gln, Asn, or Asp;
Xaa at position 82 is Gln, Asn, or Asp;
Xaa at position 118 is Gly or Leu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 139 is Gln or Glu;
said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu.

15. A protein of Claim 14, wherein R¹ is Arg.

16. A process of making a protein of any one of Claims 1 through 13, which comprises:
(a) transforming a host cell with DNA that encodes the protein of any one of Claims 1 through 13, said protein having an optional leader sequence;
(b) culturing the host cell and isolating the protein encoded in step (a); and, optionally,
(c) cleaving enzymatically the leader sequence to produce the protein of any one of Claims 1 through 13.

17. The process of Claim 16, wherein the leader sequence is Met-R₁-.

18. The process of Claim 17, wherein the leader sequence is Met-Arg-.

19. A pharmaceutical formulation, which comprises a protein as claimed in any one of Claims 1 through 13 together with one or more pharmaceutically acceptable diluents, carriers or excipients therefor.

20. A protein of any one of Claims 1 through 13 for use as a pharmaceutical agent.


SEQUENCE LIST

(1) GENERAL INFORMATION:

(i) APPLICANT:
(A) NAME: Eli Lilly and Company
(B) STREET: Lilly Corporate Center
(C) CITY: Indianapolis
(D) STATE: Indiana
(E) COUNTRY: United States
(F) POSTAL CODE (ZIP): 46285

(ii) TITLE OF INVENTION: Anti-Obesity Proteins

(iii) NUMBER OF SEQUENCES: 16

(iv) CORRESPONDENCE ADDRESS:
(A) ADDRESSEE: K. G. Tapping
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO: 1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: Protein

(ix) FEATURE:
(A) NAME/KEY: Protein
(B) LOCATION:4
(D) OTHER INFORMATION:/note= "Xaa at position 4 is Gln or
Glu;"

(ix) FEATURE:
(A) NAME/KEY: Protein
(B) LOCATION:7
(D) OTHER INFORMATION:/note= "Xaa at position 7 is Gln or
Glu;"

(ix) FEATURE:
(A) NAME/KEY: Protein
(B) LOCATION:22
(D) OTHER INFORMATION:/note= "Xaa at position 22 is Glu,
Asn, or Asp;"

(ix) FEATURE:
(A) NAME/KEY: Protein
(B) LOCATION:27
(D) OTHER INFORMATION:/note= "Xaa at position 27 is Thr
or Ala;"

(ix) FEATURE:
(A) NAME/KEY: Protein
(B) LOCATION:28
(D) OTHER INFORMATION:/note= "Xaa at position 28 is Gln,
Glu, or absent;"

(ix) FEATURE:
(A) NAME/KEY: Protein
(B) LOCATION:34
(D) OTHER INFORMATION:/note= "Xaa at position 34 is Gln
or Glu;"

(ix) FEATURE:
(A) NAME/KEY: Protein
(B) LOCATION:54
(D) OTHER INFORMATION:/note= "Xaa at position 54 is Met,
methionine sulfoxide, Leu, Ile, Val, Ala, or Gly;"

(ix) FEATURE:
(A) NAME/KEY: Protein
(B) LOCATION:56
(D) OTHER INFORMATION:/note= "Xaa at position 56 is Gln
or Glu;"

(ix) FEATURE:
(A) NAME/KEY: Protein
(B) LOCATION:62
(D) OTHER INFORMATION:/note= "Xaa at position 62 is Gln
or Glu;"

(ix) FEATURE:
(A) NAME/KEY: Protein
(B) LOCATION:63
(D) OTHER INFORMATION:/note= "Xaa at position 63 is Gln
or Glu;"

(ix) FEATURE:
(A) NAME/KEY: Protein
(B) LOCATION:68
(D) OTHER INFORMATION:/note= "Xaa at position 68 is Met,
methionine sulfoxide, Leu, Ile, Val, Ala, or Gly;"

(ix) FEATURE:
(A) NAME/KEY: Protein
(B) LOCATION:72
(D) OTHER INFORMATION:/note= "Xaa at position 72 is Glu,
Asn, or Asp;"

(ix) FEATURE:
(A) NAME/KEY: Protein
(B) LOCATION:75
(D) OTHER INFORMATION:/note= "Xaa at position 75 is Gln
or Glu;"

(ix) FEATURE:
(A) NAME/KEY: Protein
(B) LOCATION:77
(D) OTHER INFORMATION:/note= "Xaa at position 77 is Ser,
or Ala;"

(ix) FEATURE:
(A) NAME/KEY: Protein
(B) LOCATION:78
(D) OTHER INFORMATION:/note= "Xaa at position 78 is Gln,
Asn, or Asp;"

(ix) FEATURE:
(A) NAME/KEY: Protein
(B) LOCATION:82
(D) OTHER INFORMATION:/note= "Xaa at position 82 is Gln,
Asn, or Asp;"

(ix) FEATURE:
(A) NAME/KEY: Protein
(B) LOCATION:118
(D) OTHER INFORMATION:/note= "Xaa at position 118 is Gly
or Leu;"

(ix) FEATURE:
(A) NAME/KEY: Protein
(B) LOCATION:130
(D) OTHER INFORMATION:/note= "Xaa at position 130 is Gln
or Glu;"

(ix) FEATURE:
(A) NAME/KEY: Protein
(B) LOCATION:134
(D) OTHER INFORMATION:/note= "Xaa at position 134 is Gln
or Glu;"

(ix) FEATURE:
(A) NAME/KEY: Protein
(B) LOCATION:136
(D) OTHER INFORMATION:/note= "Xaa at position 136 is Met,
methionine sulfoxide, Leu, Ile, Val, Ala, or Gly;"

(ix) FEATURE:
(A) NAME/KEY: Protein
(B) LOCATION:139
(D) OTHER INFORMATION:/note= "Xaa at position 139 is Gln
or Glu;"

(xi) SEQUENCE DESCRIPTION: SEQ ID NO: 1:

Val Pro Ile Xaa Lys Val Xaa Asp Asp Thr Lys Thr Leu Ile Lys Thr
1               5                   10                  15

Ile Val Thr Arg Ile Xaa Asp Ile Ser His Xaa Xaa Ser Val Ser Ser
            20                  25                  30

Lys Xaa Lys Val Thr Gly Leu Asp Phe Ile Pro Gly Leu His Pro Ile
        35                  40                  45

Leu Thr Leu Ser Lys Xaa Asp Xaa Thr Leu Ala Val Tyr Xaa Xaa Ile
    50                  55                  60

Leu Thr Ser Xaa Pro Ser Arg Xaa Val Ile Xaa Ile Xaa Xaa Asp Leu
65                  70                  75                  80

Glu Xaa Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Xaa Tyr Ser Thr Glu Val Val Ala Leu Ser Arg
        115                 120                 125

Leu Xaa Gly Ser Leu Xaa Asp Xaa Leu Trp Xaa Leu Asp Leu Ser Pro
    130                 135                 140

Gly Cys
145

(2) INFORMATION FOR SEQ ID NO: 2:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 3:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 3:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 4:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 5:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 5:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 6:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 6:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 7:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 7:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 8:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO: 8:

Val Pro Ile Gln Lys Val Gln Asp Asp Thr Lys Thr Leu Ile Lys Thr
                5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 9:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

(xi) SEQUENCE DESCRIPTION: SEQ ID NO: 9:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 10:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 10:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Thr Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 11:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 11:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 12:

(i) SEQUENCE CHARACTERISTICS:
(A) LENGTH: 458 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 12:

CATATGAGGG TACCTATCCA AAAAGTACAA GATGACACCA AAACACTGAT AAAGACAATA 60

GTCACAAGGA TAAATGATAT CTCACACACA CAGTCAGTCT CATCTAAACA GAAAGTCACA 120

GGCTTGGACT TCATACCTGG GCTGCACCCC ATACTGACAT TGTCTAAAAT GGACCAGACA 180

CTGGCAGTCT ATCAACAGAT CTTAACAAGT ATGCCTTCTA GAAACGTGAT ACAAATATCT 240

AACGACCTGG AGAACCTGCG GGATCTGCTG CACGTGCTGG CCTTCTCTAA AAGTTGCCAC 300

TTGCCATGGG CCAGTGGCCT GGAGACATTG GACAGTCTGG GGGGAGTCCT GGAAGCCTCA 360

GGCTATTCTA CAGAGGTGGT GGCCCTGAGC AGGCTGCAGG GGTCTCTGCA AGACATGCTG 420

TGGCAGCTGG ACCTGAGCCC CGGGTGCTAA TAGGATCC 458

(2) INFORMATION FOR SEQ ID NO: 13:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 13:

GTGCTGGCCT TCTCTAAAAG TTGCAGCTTG CCACAGACCA GTGGCCTGCA GAAACCGGAA 60

AGTCTGGACG GAGTCCTGGA AGCC 84

(2) INFORMATION FOR SEQ ID NO: 14:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 14:

TGAGGCTTCC AGGACTCCGT CCAGACTTTC CGGTTTCTGC AGGCCACTGG TCTGTGGCAA 60

GCTGCAACTT TTAGAGAAGG CCAGCAC 87

(2) INFORMATION FOR SEQ ID NO: 15:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 15:

GTGCTGGCCT TCTCTAAAAG TTGCCACTTG CCAGCTGCCA GTGGCCTGGA GACATTGGAC 60

AGTCTGGGGG GAGTCCTGGA AGCC 84

(2) INFORMATION FOR SEQ ID NO: 16:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 16:

TGAGGCTTCC AGGACTCCCC CCAGACTGTC CAATGTCTCC AGGCCACTGG CAGCTGGCAA 60

GTGGCAACTT TTAGAGAAGG CCAGCAC 87